# EUROPEAN PATENT SPECIFICATION

(11) **EP 1 418 188 B1**
(45) Date of publication and mention of the grant of the patent: **11.11.2009**
(21) Application number: 04002645.2
(22) Date of filing: 25.08.2001
(51) Int. Cl.: A61L 27/52, A61L 27/16, C08F 220/56

(54) **Polyacrylamide hydrogel and its use as an endoprosthesis**
Polyacrylamidhydrogel und seine Verwendung als Endoprothese
Hydrogel de polyacrylamide et son utilisation comme endoprothèse

(30) Priority: 25.08.2000 DK 200001262
(43) Date of publication of application: 12.05.2004
(62) Divisional of application: 01960207.7
(73) Proprietor: Contura A/S, 2860 Søborg (DK)
(72) Inventor: Lessél, Robert, 2650 Brondby (DK); Petersen, Jens, 3460 Birkerod (DK); Schmidt, Richard, 2950 Vedbaek (DK); Sorensen, Jens-Erik, 2900 Hellerup (DK)
(74) Representative: Plougmann & Vingtoft A/S

(56) References cited:
- WO-A-99/10021
- RU-C- 2 127 129
- US-A- 5 658 329
- US-A- 5 798 096

## Description

### FIELD OF INVENTION

The present Invention relates to a novel polyacrylamide hydrogel cross-linked polyacrylamide. The hydrogel is obtainable by combining the acrylamide and methylene bis-aerytamide in a specific ratio so as to confer physical properties to the hydrogel. The present invention further relates to the use of the hydrogel for the preparation of an endoprosthesis for cosmetic surgery, reconstructive surgery as a soft-tissue filler endoprosthesis.

### BACKGROUND OF THE INVENTION

Natural and synthetic polymers such as collagen, soya, glycerol, silicone, polyvinylpyrolidone and hyaluronic acid have been utilised as enddprostheses. Materials used for endoprostheses generally try to imitate the natural soft tissue and are intended to be safe to the health of the patient.

Polyacrylamide gels have also been disclosed. US 5,798,096 relates to a blocompatible hydrogel containing 3.5 to 6.0% cross-linked polyacrylamide. However, US 5,798,096 teaches that concentrations below 3.5% make the hydrogel unstable.

GB 2114578 relates to a polyacrylamide gel for medical and biological purposes containing 3 to 28% polyacrylamide with the remainder of the mass of the gel comprised of a physiological solution.

US 5,658,329 relates to an implantable endoprosthesis comprising a shell filled with a polyacrylamide gel comprising 2 to 20% polyaaylamide by weight and a viscosity range of 15 to 75 Pas.

Formecryl® polyauytamide is a soft-tissue endoprosthesis consisting of 5% reticulated polyacrylamide polymer and 95% apyrogenic water commerdalised as an injectable device for medical and dental use to correct congenital or acquired deficits such as wrinkles, lines and scars. It is to be implanted with a syringue in the hypodermis.

US 5,308,404 relates to a process for preparing polyacrylamide gel plates for electrophoresis.

WO 99/10021 relates to an injectable, biocompatible hydrogel comprising 0.5 to 10% polyacrylamide and an antiblotic or antiseptic. WO 99/10021 is directed to the solving the problem of sappuration and rejection of the gel in its use an endoprosthesis.
RU 2122129 discloses the preparation of a gel-like material for plastics of soft tissues wherein the material contains a copolymer prepared by combining acrylamide and methylene-bis-acrylamide in aqueous media at pH 9.0- 9.5 at 20-90°C followed by heating to 100-105°C for 2-4 hours.

### SUMMARY OF THE INVENTION

The bio-stable hydrogel typically has a molecular weight between 0.01 x 10⁶ and 20 x 10⁶. The polymer is resistant to biological degradation and is not permeable through biological membranes. The polyacrylamide hydrogel of the invention Is fully biocompatible (according to ISO standard test ISO-10993). The polyacrylamide hydrogel does not have cytotoxic effect on human fibrobiasts, is non-toxic, non-carcinogenic, non-allergenic, non-mutagenic, and resistant to enzymatic and microbiological degradation. Furthermore, the polymer is not water-soluble. The hydrogel is useful as an endoprosthetic amount, said gel tailored to the defect it seeks to correct.
The invention is directed to the use of a hydrogel obtainable by the steps comprising combining acrylamide and methylene bis-acrylamlde in amounts so as to give less than 3.5% by weight polymer, based on the total weight of the hydrogel; radical initiation; and washing with pyrogen-free water or saline solution; for the preparation of an endoprosthesis for soft tissue filling by injection in a mammal, for the preparation of an endoprosthesis for cosmetic or functional defect of the face or body by injection, or for the preparation of a prosthetic device for the treatment of cosmetic or functional defect in lips by injection, the hydrogel having a concentration of acrylamide and N,N'-methylene-bis-acrylamide-below 50 ppm; and wherein the hydrogel has a complex viscosity of 5 to 90 Pas.

### DETAILED DESCRIPTION OF THE INVENTION

### Hydrogels and Their Preparation

The success of plastic or reconstructive surgery depends to a great extent on the physical properties of the materials utilized. They must most certainly be biocompatible, stable and non-toxic but they must also have physical properties that mimic the bodily tissue they are replacing, as in reconstructive surgery, or mimic the bodily tissue in the proximity of the endoprosthesis, as In cosmetic surgery.

Materials such as collagen are resorbed into the body over short periods of time. Silicone and Soya have encountered serious safety issues. There is currently a need for a safe, stable, biocompatible material that possesses the physical properties to mimic soft tissue.

The present inventors have surprisingly found that a polyacrylamide hydrogel comprising less than 3.5% polyacrylamide, based on the total weight of the hydrogel, is an effective endoprosthosis with advantageous physical properties. Contrary to US 5,798,096, the polyacrylamide gel is stable. The hydrogel, as prepared by a process according to the present invention, is cross-linked with methylene bis-acrylamide to a degree such that the endoprosthesis prepared from said hydrogel possesses advantageous physical characteristics. The hydrogel according to the present invention is a new chemical entity as indicated by its novel and advantageous physical characteristics. These secondary characteristics are indicative that the degree of cross-linking in the hydrogel of the present invention differs greatly from polyacrylamide hydrogels prepared by disclosed processes. This degree of cross-linking is a critical contributor to its physical properties.

The present investigators have provided a bio-stable hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution. The bio-stable hydrogel typically has a molecular weight between 0.01 x 10⁶ and 20 x 10⁶. The polymer is resistant to biological, degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carclnogenic, non-allergenic, non-mutagenic, and resistant to enzymatic and microbiological degradation. Furthermore, the polymer is not water-soluble. A primary object of the invention is to provide a hydrogel comprising less than 3.5% polyacrylamide by weight, based on the total weight of the hydrogel, said hydrogel obtainable by combining acrylamide and methylene bis-acrylamide, radical initiation, and washing with pyrogen-free water or saline solution; said hydrogel being blocompatible, and said combining being in a molar ratio of 150:1 to 1000:1.

A primary object of the invention is to provide a hydrogel comprising less than 3.5% polyacrylamide by weight, based on the total weight of the hydrogel, said hydrogel obtainable by combining acrylamide and methylene bis-acrylamide, radical initiation, and washing with pyrogen-free water or saline solution; said hydrogel being blocompatible, and said combining being in a molar ratio of 150:1 to 1000:1.

Alternatively defined, one aspect of the invention relates to hydrogel comprising i) less than 3.5 % polyacrylamide by weight, based on the total weight of the hydrogel, cross-linked with methylene bis-acrylamide and ii) at least 95% pyrogen-free water or saline solution.

A further aspect of the Invention relates to a hydrogel for use as an injectable or implantable endoprosthesis said hydrogel comprising i) less than 3.5% polyacrylamide by weight, based on the total weight of the hydrogel, cross-linked with methylene bis-acrylamide and ii) at least 95% pyrogen-free water or saline solution.

In all embodiments wherein the hydrogel comprises less than 3.5% polyacrylamide, by weight, based on the total weight of the hydrogel, the hydrogel typically further comprises at least 95% pyrogen-free water or saline solution.

The hydrogel comprises less than 3.5% polyacrylamide preferably comprising at least 0.5%, such as at least 1 %, preferably at least 1.5% polyacrylamide, such as at least 1.6% polyacrylamide by weight, based on the total weight of the hydrogel.

The hydrogel comprising less than 3.5% polyacrylamide are chemically stable and biostable but may be very fluid such that they may be **characterised in that** it has a complex viscosity not less than 2 Pas, such as not less than 3, 4 or 5 Pas. In a suitable embodiment, the hydrogel comprising less than 3.5% polyacrylamide is characterised in that it has complex viscosity from about 2 to 90, such as 5 to 80 Pas, preferably from about 6 to 76, such as from about 6 to 80, 6 to 40, 6 to 20, such as 6 to 15 Pas.

The hydrogel comprising less than 3.5% polyacrylamide has elastic properties in that the hydrogel may be characterised in that it has elasticity module of not less than 10 Pa, such as not less than 20, 25, 30, 31, 32, 33, 34 or 35 Pa, such as not less than 38 Pa. Typically, the hydrogel has an elasticity module from about 10 to 700 Pa, such as about 35 to 480 Pa.

These rheological features are in due in part to the degree of cross-linking and to the degree of swelling of the hydrogel. The hydrogel comprising less than 3.5% polyacrylamide may be characterised in that the cross-linked polyacrylamide is to such as degree so as to have an efficient cross-linking density of about 0.2 to 0.5%, preferably about 0.25 to 0.4%.

The cross-linking density is in turn due In part to the molar ratio between the acrylamide and mothylone-bis-acrylamide. Typically said ratio is In the range 175:1 to 800:1, such as from 225:1 to 600:1, preferably from 250:1 to 550:1, most preferably from 250:1 to 500:1. The absolute and relative amount of the redox agent (TEMED) and the initiator also influence the degree of cross-linking, As can be seen from Tables 1, 2, 3, 4, the present investigators have adjusted these parameters to influence the rheological properties of the hydrogel.

The biocompatible hydrogels of the invention comprising less than 3.5% polyacrylamide may be suitably characterised, at least In part, by one or more of the following features: i) a cross-linking density of 0.2% to 0.5 %; ii) an elasticity modulus (G') of 10 to 700 Pa; iii) a complex viscosity of 2 to 90 Pa s; iv) a dry matter content of less than 3.5% such as less than 3.4, such as less than 3.3, such as less than 3.2, such as less than 3.1, such as less than 3.0, such as less than 2.9, such as less than 2.8, such as less than 2.7, such as less than 2.6 Pa s; v) a refractive Index between 1.33 and 1.34.

As stated, in all aspects of the invention wherein the hydrogel comprises less than 3.5% polyacrylamide by weight, based on the total weight of the hydrogel, the hydrogel further comprising at least 95% pyrogen-free water or saline solution. In all embodiments wherein the hydrogel further comprises saline solution, the hydrogel preferably comprises less than 3 % polyacrylamide by weight, based on the total weight of the hydrogel.

The hydrogel of the invention Is substantially free of materials which contribute to the solid weight content other than the acrylamide, methylene-bis-acrylamide and residual amounts (if any) of the initiators. The hydrogel is substantially free of any other polymeric content. The hydrogel further comprises at least 75% by weight pyrogen-free water or saline solution, preferably pyrogen-free water. In a suitable embodiment of the invention, the hydrogel comprises at least 80% by weight pyrogen-free water or saline solution, preferably at least 85 %, more preferably at least 90%, even more preferably at least 95% by weight pyrogen-free water or saline solution.

The hydrogel comprises pyrogen-free water or saline solution. The combining of the reagents and the casting of the gel may therefore be done in pyrogen-free water or saline solution. The use of saline solution will clearly increase the total solid weight content of the hydrogel but does not significantly influence the polyacrylamide content during the polymerisation reaction.

A suitable saline solution has an osmolarity similar to that of interstitial fluid. Suitable saline solutions include but are not limited to the group selected from 0.25- 1% aqueous sodium chloride, a Ringer-Lockart solution, an Earte solution, a Hanks solution, an Eagle medium, a 0.25 - 1 % glucose solution, a potassium chloride solution, and a calcium chloride solution. In a preferred embodiment, the saline solution is an about 0.8- 1 % aqueous sodium chloride solution, such as a 0.8, 0.9 or 1 % aqueous sodium chloride solution.

As stated, pyrogen-free water or saline solution is used for the washing process. The washing process serves, In part, to remove all but trace amounts of the monomers acrylamide and N,N'-methylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, most preferably below 20 ppm, typically below 10 ppm, typically below 5 ppm. In the method of the invention, the washing step comprises swelling the product for 50 to 250 hours, more typically for 70 to 200 hours.

The hydrogel comprising less than 3.5% polyacrylamide was surprisingly found to be stable at very low solid weight content, contrary to the teachings of US 5,798,096. Polyacrylamide hydrogels with a solid-weight content of 0.5% have been prepared by the present investigators. Preferred embodiments of the hydrogel of the invention comprise at least 0.5%, such as at least 1 %, preferably at least 1.5% polyacrylamide, such as at least 1.6% polyacrylamide by weight, based on the total weight of the hydrogel.

The hydrogel is suitably a composite of cross-linked polyacrylamide chains and pyrogen-free water. Water contained in the hydrogel as part of a composite is loosely bound with the polymer chains. When present in a body, some of the water molecules move into the tissue by osmosis resulting in a smoothing out of the affected skin surface. In the embodiment wherein the hydrogel comprises saline solution, the iso-osmolarity of the saline solution with interstitial fluid minimises immune responses.

As mentioned, the physical properties of the hydrogel are influenced in part by the degree of aoss-linking. The degree of cross-linking may be controlled in pad by the molar ratio of cross-inking agent, methylene bis-acrylamide, to acrylamide.

The hydrogen comprises at least 0.5%. Preferably such as at least 1 %, preferably at least 1.5% polyacrylamide, such as at least 1.6% polyacrylamide by weight, based on the total weight of the hydrogel.

In a especially preferred embodiment of the invention, the hydrogel is obtainable by combining acrylamide and methylene bis-acrylamide in amounts so as to give 1.6 to 3.5% by weight polyacrylamide and in a molar ratio of 150:1 to 1000:1, radical initiation, and washing with pyrogen-free water or saline solution.

The combining of acrylamide and methylene bis-acrylamide in preferably done in a molar ratio between acrylamide and methylene bis-acrylamide is from 175:1 to 800:1, such as from 225:1 to 600:1, preferably from 250:1 to 550:1, most preferably from 250:1 to 500:1.

An illustrative preparation of the hydrogel according to the present invention is described in Example 1. The hydrogel having the desired physical properties has been obtained by combining acrylamide and methylene bis-acrylamide in a ratio of about 250:1, such as 252:1, 254:1, 256:1, 258:1 and 260:1, as well as by combining acrylamide and methylene bis-acrylamide in a ratio of about 500:1, such as 498:1, 496:1 494:1 492:1, or 490:1. The hydrogel according to the present invention preferably has a complex viscosity from about 2 to 90, such as 5 to 80 Pas, typically from about 6 to 76, such as from about 6 to 60, 6 to 40, 6 to 20, such as 6 to 15 Pas. In a suitable embodiment, the washing step comprises swelling the product of the radical initiation step until the complex viscosity is from about 6 to 100 Pas.

In Suitable embodiment of the invention, the hydrogel has a degree of cross-linking such that it has complex viscosity of not less than 2 Pas, such as not less than 3. 4 or 5 Pas, such as not less than 5.5 Pas, such as not less than 6 Pas, preferably not less than 6.2 Pas.

The elasticity module is an alternative physical characteristic of the hydrogel indicative, in part, of the degree of cross-linking of the hydrogel according to the present invention. Typically the degree of cross-linking is such that the hydrogel has an elasticity module of not less than 10 Pa, such as not less than 25, 30, 31, 32, 33, 34 or 35 Pa, such as not less than 38 Pa. The get may be **characterised in that** it has elasticity module from about 10 to 700 Pa, such as about 35 to 480 Pa.

As stated, in a most preferred embodiment, the hydrogel is obtainable by combining acrylamide and methylene bis-acrylamide in amounts so as to give 1.6 to 3.5% by weight polyacrylamide and in a molar ratio of 150:1 to 1000:1, radical initiation, and washing with pyrogen-free water or saline solution. The radical initiation step yields a hydrogel still comprising toxic reagents and not yet possessing the beneficial physical properties of the hydrogel of the present invention. The washing step comprises swelling the hydrogel produced from the radical initiation step until the complex viscosity is from about 2 to 90 Pas.

Alternatively measured, the washing step may comprise swelling the product of the radical initiation step until the elasticity module is elasticity module from about 10 to 700 Pa, such as from about 35 to 480 Pa.

By nature, a low degree of cross-linking typically results in a higher swelling rate consequently lowering the dry matter content (percentage acrylamide), as well as lowering the elasticity module and viscosity. Thus, in addition to the degree of cross-linking, the time the hydrogel is exposed to the washing step influences to a degree the physical properties of the gel.

Typically, the washing step comprises swelling the product for about 80 to 100 hours, such as 90-95 hours. This usually results in an increase in weight of the hydrogel of about 75 to 150%, usually around 100% increase.

Furthermore, the amounts of the free-radical initiator in the radical initiation step and the amount of co-initiator influences the chain length and thus the physical properties of the hydrogel. In a typical preparation of the hydrogel, N-,N-,N-,N-tetramethyl ethylene diamine (TMED) is used as the co-initiator and ammoniumpersulfate (APS) is used as the free-radical initiator (redox-system). Adequate amounts of initiator and co-initiator are required to obtain the hydrogel according to the invention. As an illustration, an insufficient amount of these reagents will result in shorter chain lengths and consequently influence the degree of cross-linking and hence the physical properties of the hydrogel. Other reaction conditions, such as temperature also influence chain length.

As stated, the degree of cross-linking influences the physical properties of the hydrogel. The degree of cross-linking of the hydrogel of the present invention may be indirectly measured, as described above, by the elasticity module and/or by the complex viscosity. An alternative measure of the degree of cross-linking of the hydrogel is its efficient coss-linking density. The hydrogel of the present invention preferably comprises 1.6 to 3.5% (wt/wt) polyacrylamide cross-linked with methylene bis-acrylamide and ii) pyrogen-free water or saline solution, preferably such that the degree of cross-linking as measured by its efficient cross-linking density is such that the efficient cross-linking density is about 0.2 to 0.5%, preferably about 0.25 to 0.4%.

In a suitable embodiment of the present invention, the hydrogel may comprise 1.6 to 3.25% (wt/wt) polyacrylamide, such as 1.8 to 3.1, 2.0 to 3.0, 2.0 to 2.9, preferably 2.0 to 2.8 (wt/wt) polyacrylamide.

### Medical Use of Polyacrylamide Hydrogels

The hydrogels of the invention are intended for use as endoprosthetic devices. The endoprosthetic devices of the invention and methods of treatment described herein may use the hydrogel in any of the embodiments described supra.

The endoprosthetic device may be administered to the body of an individual by means of injection, such as through a syringe or catheter. The hydrogel injected into an affected area is obtainable by combining acylamide and methylene bis-acryatmide in amounts so as to give 1.6 to 3.5% by weight polyacrylamide and in a molar ratio of 150:1 to 1000:1, radical initiation, and washing with pyrogen-free water or saline solution. The ratio of acrylamide to methylene bis-acrylamide is preferably such that the complex viscosity is from about 2 to 90, such as 5 to 80 Pas, preferably from about 6 to 76 Pas or such that the elasticity module is from about 10 to 700 Pa, such as about 35 to 480 Pa. The washing step may be done to the extent such that the complex viscosity is from about 6 to 80 Pas or such that the elasticity module is from 10 to 700 Pa, such as about 35 to 480 Pa.

The cosmetic and reconstructive surgery may be for facial correction such as for altering the form or size of lips, to treat wrinkles or facial asymmetries, or altering the shape of a nose as well as a variety of facial corrections known to the person skilled in the art.

Furthermore, cosmetic and reconstructive surgery may be to correct defects caused by trauma or due to diseases such as, for instance, hemiphlegia.

An object of the present invention is an injectable endoprosthesis comprising i) 1.6 to 3.5% by weight polyacrylamide cross-linked with methylene bis-acxytamide and ii) pyrogen-free water or saline solution. An injectable endoprosthesis according to the present invention preferably has a viscosity complex from about 2 to 90, such as 5 to 80 Pas, preferably from about 6 to 76, such as from about 6 to 60, 6 to 40, 6 to 20, such as 6 to 15 Pas.

Wherein the endoprosthesis is injected, the hydrogel is generally somewhat fluid in nature.

The solid-weight content of weight percentage acrylamide, as measured after the washing step, as well as the degree of cross-linking is adapted according to the use of the endoprosthesis prepared from the hydrogel. In preferred embodiments, the endoprosthesis is preferably prepared from a hydrogel comprising 1.6 to 3.25% (wt/wt) polyacrylamide, such as 1.8 to 3.1, 2.0 to 3.0, 2.0 to 2.9, preferably 2.0 to 2.8 (wt/wt) polyacrylamide. Alternatively defined, the degree of cross-linking of the hydrogel for use as an endoprosthesis may preferably be such that the complex viscosity of the hydrogel is from about 2.0 to 15 Pas such as from about 5.5 to 15 Pas, such as from 6 to 12 Pas. Alternatively measured, for facial corrections the degree of cross-linking of the hydrogel are preferably such that the elasticity module is from about 10 to 100 Pa, such as about 35 to 75, particularly 35 to 60 Pa, such as 35 to 50 Pa.

The endoprosthesis may be to correct a defect which is the result of trauma such as tumours or physical injury as well as congenital defects.

In an alternative embodiment of the invention, the endoprosthesis may comprise a medicament for use in therapy.

### Polyacrylamide Hydrogel as a Soft-Tissue Filler Endoprosthesis

The term "facial" in this aspect of the invention intended mean of relation to all areas of the face, such as but not exclusively, the cheeks, the jaw, the neck, the forehead, under the eyes, the head area, and the nose.

The term "body contouring" is intended to mean cosmetic or reconstructive surgery wherein soft tissue is augmented in order to correct a cosmetic or non-cosmetic defect in the soft tissue in the body, excluding the face, lips, breasts and penis. In this aspect of the invention, the endoprosthesis is directed to facial corrections, lip augmentation, and body contouring.

In this aspect of the invention, the term hydrogel relates to the polyacrylamide polymer of the invention comprising less than 3.5% polyacrylamide and at least 95% pyrogen-free water or saline solution whereas the term endoprosthesis relates to the hydrogel present in the body.

The polyacrylamide hydrogel is obtainable by the polymerisation of the monomers acrylamide and N,N'-methylene-bis-acrylamide under radical initiation, followed by washing of the polymer with pyrogen-free water or saline solution. The washing of the polymer results in a swelling of the gel, due to absorption of the pyrogen-free water or saline solution by the polymer. The swelling of the hydrogel influences the solid weight content of the gel, i.e. the amount of polymeric material, polyacrylamide. The solid weight content of the hydrogel influences, at least in part, the physical (rheological) properties of the hydrogel and thus the ability to mimic human tissue when used as an endoprosthesis.

The present investigators have prepared a hydrogel having the desired rheological properties to act as a soft tissue filler endoprosthesis which is completely atoxic, stable, and non-resorbable. The present investigators have developed the hydrogel to be particularly amenable for use as an endoprosthesis for facial cosmetic or reconstructive surgery, for body contouring and for lip augmentation or reconstruction. The hydrogel in this aspect of the invention is not directed for use as an endoprosthesis for breast or penis augmentation.

A first aspect of in this aspect of the invention relates to a hydrogel for use as a soft tissue filler endoprosthesis said hydrogel obtainable by combining acrylamide and methylene bis-acrylamide in amounts so as to give less than 3.5% by weight polyacrylamide, based on the total weight of the hydrogel; radical initiation; and washing with pyrogen-free water or saline solution. Typically, the hydrogel is obtained by said combining acrylamide and methylene bis-acrylamide in a molar ratio of 150:1 to 1000:1. The hydrogel obtained In this manner has a structural formula as shown in Figure 1, is sterile, has transparent or colourless appearance and has a pH in the range of 6.5 to 9.0, typically 7.0 to 9.0. Furthermore the hydrogel of the invention is stable to oxygen, high pressure, high and low temperatures, enzymes and bacteria.

This aspect of the invention thus relates to the use of a hydrogel comprising less than 3.5% by weight polyacrylamide, based on the total weight of the hydrogel, for the preparation of an endoprosthesis for soft tissue filling. Given the hydrogel in this aspect of the invention is directed for use as an endoprosthesis, it must be stable. Furthermore, given the hydrogel of the invention is directed for use as an endoprosthesis for selected parts of the human anatomy, the hydrogel typically comprises at least 0.5 % by weight polyacrylamide, based on the total weight of the hydrogel, preferably at least 1.0 % by weight polyacrylamide, more preferable at least 1.5 % by weight polyacrylamide, such as at least 1.6 % by weight polyacrylamide, based on the total weight of the hydrogel. Typically, the hydrogel of the present invention may have a solid weight content of 1.5, 1.6, 1.7 1.8, 1.9, 2.0, 2.1, 2.2, 2.3, 2.4, 2.5, 2.6, 2.7, 2.8, 2.9, 3.0, 3.1, 3.2, 3.3, 3.4, or 3.5% polyacrylamide, based on the total weight of the hydrogel.

In a preferred embodiment of this aspect of the invention, the hydrogel comprises about 1.9 to 2.9 % by weight polyacrylamide, based on the total weight of the hydrogel. The hydrogel typically further comprises at least 95 % by weight pyrogen-free water or saline solution, preferably pyrogen-free water. In a preferred embodiment, the hydrogel comprises at least 96 % by weight pyrogen-free water or saline solution, preferably pyrogen-free water, more preferably at least 97 % by weight pyrogen-free water or saline solution, preferably pyrogen-free water, such as 95 %, 95.5 %, 96 %, 96.5 %, 97 %, or 97.5 % by weight pyrogen-free water or saline solution, preferably pyrogen-free water

A suitable saline solution has an osmolarity similar to that of interstitial fluid. Suitable saline solutions include but are not limited to the group selected from 0.25- 1% aqueous sodium chloride, a Ringer-Lockart solution, an Earle solution, a Hanks solution, an Eagle medium, a 0.25 - 1 % glucose solution, a potassium chloride solution, and a calcium chloride solution.. In a preferred embodiment, the saline solution is an about 0.8-1 % aqueous sodium chloride solution, such as a 0.8, 0.9 or 1 % aqueous sodium chloride solution.

Pyrogen-free water or saline solution is used for the washing process. The washing process serves, in part, to remove all but trace amounts of the monomers acrylamide and N,N'-mothylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, particularly preferably below 20 ppm, typically below 10 ppm, most preferably less than 5 ppm.

The solid weight content of the hydrogel of the present invention is essentially completely contributed by the polyacrylamide with residual contribution by the initiator and N,N'-metylene-bis-acrylamide. The hydrogel is substantially free of any other polymeric content.

As stated, the hydrogel of the invention is biocompatible, atoxic, non-allergenic, non-resorbable, chemically inert and stable to oxygen, high pressure, high and low temperatures, enzymes and bacteria. In the event that the hydrogel is exposed to excessive amounts of UV light, the physical features of the hydrogel are altered and is converted into a glue-like substance. Advantageously, this substance is also non-toxic.

Upon administration of the hydrogel, a thin layer of connective tissue surrounds the endoprosthesis, enabling the endoprosthesis to become a stable part of the connective tissue. Due to the blo-stabllity of the hydrogel and the thin layer of connective tissue, the endoprosthesis may be easily removed from the patient when placed in the subcutaneous area. This advantage is at least in part due to the stability of the hydrogel which in turn is at least in part due to the washing process.

Several factors affect the rheological properties of the hydrogel, such as the relative amount of monomer used, the relative amount of initiator, the temperature, the time of polymerisation, and other parameters of the polymerisation process, as well as the washing process. Thus, the polymerisation process may provide a hydrogel with an array of viscosities having a solid weight content of less than 3.5%. The invention is directed to a hydrogel as a soft-tissue filler endoprosthesis and thus the hydrogel preferably has a viscosity tailored to the soft-tissue it is intended to mimic. The hydrogel of the Invention is typically for use as an injectable endoprosthesis for cosmetic or reconstructive surgery of the face, cosmetic or reconstructive surgery of the body (body contouring), and augmentation or reconstructive surgery of the lips.

The hydrogel of this aspect of the invention is injectable.

In one embodiment of this aspect of the invention, the endoprosthesis is for use in facial cosmetic or reconstructive surgery and the hydrogel has a complex viscosity of about 2 to 100 Pas, preferably about 5 to 90 Pas, such as about 5 to 60 Pas, such as about 10 to 260 Pas. Most preferably, the endoprosthesis for use in facial cosmetic or reconstructive surgery was place by means of injection of the hydrogel.

Depending on the condition and region of the epidermis of the face where the soft-tissue filler is required (e. g. chin as opposed to cheek), the viscosity of the hydrogel may vary. Accordingly, the hydrogel may be for use in facial cosmetic or reconstructive surgery and have a complex viscosity of about 2 to 20 Pas, preferably about 2 to 18 Pas, such as about 2 to 15 Pas or 2 to 10 Pas, more preferably 2 to 7 Pas, most preferably 3 to 5 Pas.

In typical embodiments, the endoprosthesis may be for uses in correction of facial contour deformities due to ageing, acne, trauma, surgery, infection or congenital deformities. The facial features typically in need of correction are, for instance, the cheekbones, nasolabial folds, glabellar frowns, depressed contours of the mouth, the chin, the size or shape the lips, as well as other soft tissue deficiencies of the face. The hydrogel restores the skin contours correcting soft tissue contour deformities of the face such as wrinkles and folds. The hydrogel may serve for the preparation of an injectable hydrogel for lip enhancement or correct an array of aesthetic defects caused by congenital, traumatic or ageing alterations.

As stated, this aspect of the invention relates to the use of the hydrogel for the preparation of an endoprosthesis for the filling of soft tissue filling selected from the soft tissue of the face and lips and soft tissue of the body. The hydrogel of the invention directed for use in the cosmetic or reconstructive surgery of the body (body contouring), preferably has a complex viscosity of about 5 to 50 Pas, preferably about 7 to 40 Pas, most preferably about 7 to 30 Pas.

In the embodiment wherein the hydrogel is used in the preparation of an endoprosthesis for lip augmentation or lip reconstruction the hydrogel preferably has a complex viscosity of about 2 to 10 Pas, more preferably 2 to 7 Pas, most preferably 3 to 5 Pas.

A further object of this aspect of the invention is to provide a prosthetic device for soft tissue augmentation said device being injectable and comprising a polyacrylamide hydrogel said hydrogel being obtainable by combining acrylamide and methylene bis-acrylamide in amounts so as to give less than 3.5% by weight polyacrylamide, based on the total weight of the hydrogel; radical initiation; and washing with pyrogen-free water or saline solution.

The prosthetic device of this aspect of the invention preferably comprises a hydrogel comprising at least 0.5 % by weight polyacrylamide, based on the total weight of the hydrogel, preferably at least 1 % by weight polyacrylamide, more preferable at least 1.5 % by weight polyacrylamide, such as at least 1.6 % by weight polyacrylamide, based on the total weight of the hydrogel. Typically, the hydrogel comprises about 1.9 to 2.9 % by weight polyacrylamide, based on the total weight of the hydrogel. The prosthetic device comprises the hydrogel which typically comprises at least 95% by weight pyrogen-free water or saline solution, preferably pyrogen-free water.

A further aspect of the invention relates to a method of filling soft tissue comprising administering an endoprosthesis wherein the endoprosthesis comprises a hydrogel comprising less than 3.5% by weight polyacrylamide, based on the total weight of the hydrogel. The hydrogel may be as described supra.

In an alternative embodiment of the invention, the prosthetic device comprises calls, such as stem cells. Polyacrylamide provides an excellent template and matrix for cell growth. Although the hydrogel of the invention allows, in itself, for a thin layer of connective tissue from the body of the patient to surround the device, the use of cells in combination with the hydrogel of the invention for the preparation of the device allows for cellular engraftment to the surrounding tissue.

The method of this aspect of the invention typically comprises administering the hydrogel of the invention by injecting the hydrogel, into the subcutaneous layer of the skin in the embodiments wherein the endoprosthesis is for facial cosmetic or reconstructive surgery or body contouring. In the embodiment wherein the endoprosthesis is for lip augmentation or lip reconstruction, the injecting is above the muscle tissue of the lip.

As stated, an alternative embodiment of this aspect of the invention comprises administering the hydrogel of the invention in combination with cells, such as stem cells to allow for cellular engraftment of the prosthetic device.

The method of this aspect of the invention may comprise of more than one injection so as to cover the desired area or to achieve the desired affect. The gel to be injected is typically stored in a syringe suitable for injecting the required amount for a single session treatment. Depending on the afflicted area, the amount of gel and thus volume of the syringe may vary, such as a syringe with a volume of 0.25-25 mL, such as a syringe selected from 0.5 mL, 0.7 mL, 1.0 ml, 1.5 mL. 2.0 mL, 2.5 mL, 5.0 mL, 7.5 mL, 10 mL, 12.5 mL, 15 mL, 20 mL and 25 mL. Obviously, the prosthetic device for use in facial surgery or lip augmentation be provided in a syringe in an array of volumes, typically lower in volume than the volumes provided for by the prosthetic device for body contouring. For instance, the device for lip augmentation may be provided in volumes of 0.5 ml or 0.7 mL or 1 mL whereas the device for body contouring may be provided in volumes of 2 mL, 5 mL, or 10 mL. These are purely illustrative examples and are not intended to limit the scope of the invention in any way - the device of the invention may be provided in any volume required to perform the method.

As stated, the hydrogel is highly biocompatible. The method of the invention does not comprise of adding an antibiotic, analgesic or anti-inflammatory agent to the hydrogel.

In the preferred embodiment wherein the method comprises the injection of the endoprosthesis, the injection comprises the use of a syringe with a thin gauge needle such as a 21-29 G needle. The necessary amount of the gel is injected subcutaneously in a retrograde manner by injecting the gel while withdrawing the needle. After the injection is performed, a light manipulation may be required in order to obtain an even distribution of the gel. Post-operative oedema may be treated with local packing of ice and mild pain and redness may be experienced during the first 2-3 days after injection.

In the embodiment wherein the method comprises injection of the endoprosthesis for lip augmentation or facial correction the needle of the syringe is typically particularly thin, such as a 25-29 G needle. For body contouring, the needle of the syringe may be in the range 21-23 G.

The invention is further disclosed by the Examples which are not intended to be limiting in any way

### EXAMPLES

### Example 1

### Preparation of Hydrogel

The gel is a polyacrylamide gel manufactured by a polymerisation of the monomers of acrylamide and N,N'-methylene-bis-acrylamide. The finished product may have different viscosities.

The hydrogel typically contains approximately 95% water. The concentration of the monomers acrylamide and N,N'-methylene-bis-acrylamide has been shown to be less than 10 ppm and is adequate for the desired stability of the final product, often less than 5 ppm.

The finished product must conform with respect to pH, absence of heavy metals, refractive index, stability, absence of pyrogens, and must be sterile, practically inert, and be substantially free of monomers.

### Preparation 1.1

The synthetic preparation suitably involves the following operations:
1. Two mixtures, A1 and A2, are prepared. A1 comprises water, acrylamide, N,N'-methylene-bis-acrylamide, N,N, N',N'-tetramethylene-ethylene-diamine (TEMED). A2 comprises water and ammonium persulphate;
2. The two mixtures are combined in the following ratio: 1990 mL of A1 and 10 mL of A2 and kept at 45 °C and degassed with nitrogen for 20 seconds;
3. The reaction mixture is cast into several 100 mL beakers;
4. Polymerisation is allowed to occur for 0.5 to 1.5 hours;
5. The gel is demolded;
6. Residual monomers are extracted and with equilibration in WFI water for 92 hours, changing the water several times, typically 8 times during the 92 hours;
7. The purified gels are homogenised by grinding with an vertically oscillating grid;
8. The syringe is filled with the homogenised get material;
9. Autodavation of the syringe

A typical method for preparing the hydrogel may be summarised as:

### Preparation 1.2

*Process summary*: The gel is prepared by mixing an aqueous monomer solution of acrylamide (AM) and N, N'-methylene-bis-acrylamide (BISAM) as cross-linker with N,N,N',N'-tetramethylene ethylene diamine (TMED) as co-initiator and ammoniumpersulfate (APS) as free-radical initiator (redox-system). By degassing a bulk solution with nitrogen polymerisation starts. After final polymerisation the gel transferred into a washing tank with net trays onto which the gel is placed. During water washing the gel swells and monomer residues are extracted. The swollen gel is fed and evacuated in a filling unit having the gel delivered in a syringe, which is autoclaved.

Two alternate formulations have been prepared, a lower- and a higher- end viscosity formulation. Both formulations have a solid weight content of less than 3.5% and a complex viscosity in the range of 2 to 50 Pa s, typically between 3 and 20 Pa s.

**Table 1**

| Chemical constituent | lower end viscosity | higher end viscosity |
|---|---|---|
| acrylamide | 502 g | 547 g |
| N,N'-methylene-bis-acrylamide | 2,2 g | 4,6 g |
| TMED | 3,0 g | 2,6 g |
| APS | 5,4 g | 5,0 g |
| Non-pyrogenic water | Add 10 litre | Add 10 litre |

The above are typical preparations of the hydrogel and may be adjusted within certain ranges.

### Preparation 1.3

### Polyacrylamide formulations from inline cross-linking process

A particularly interesting method of preparing the hydrogels of the invention involves an inline cross-linking process. Two Individual and eventually degassed flows, one being a pre-mix of acrylic amide, bis-methylene acryl amide (the cross-linker) and TEMED, the other being the AMPS initiator solution, are pumped into a static mixer for mixing, chemical initiation and subsequent extrusion downstream into a pipe reactor made of Teflon or steel in which the polymerisation occurs. Washing of the gel is simplified due to high surface area of gel from reactor.

By selecting monomer, cross-linker and initiator concentrations and their relative molar ratios, and by regulating the two flow rates and the polymerisation temperatures, it is possible to produce gels that are varying in degree of crosslinking and in solids content.

### Preparation 1.4

The reagents were combined in ratios described in Tables 2, 3 and 4, and washed as described in the Tables (with pyrogen-free water unless indicated otherwise) to give low, medium, and high viscosity formulations. Hydrogels with solid weight contents between 0.5 and 25% polyacrylamide were prepared.

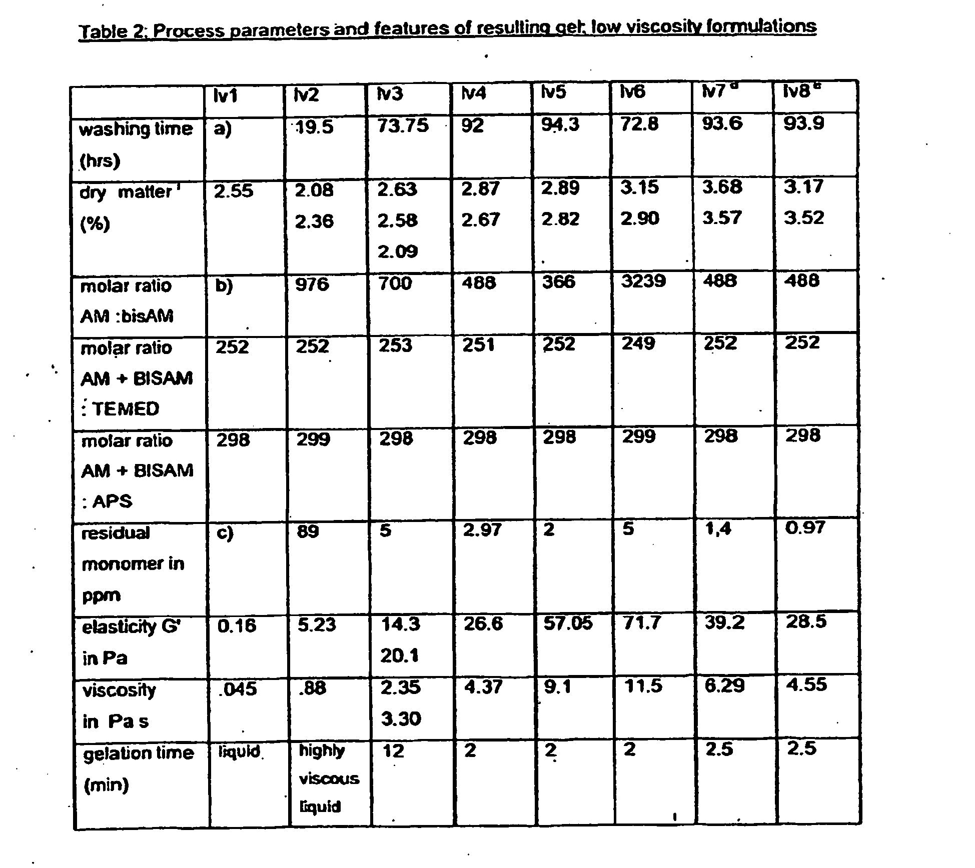

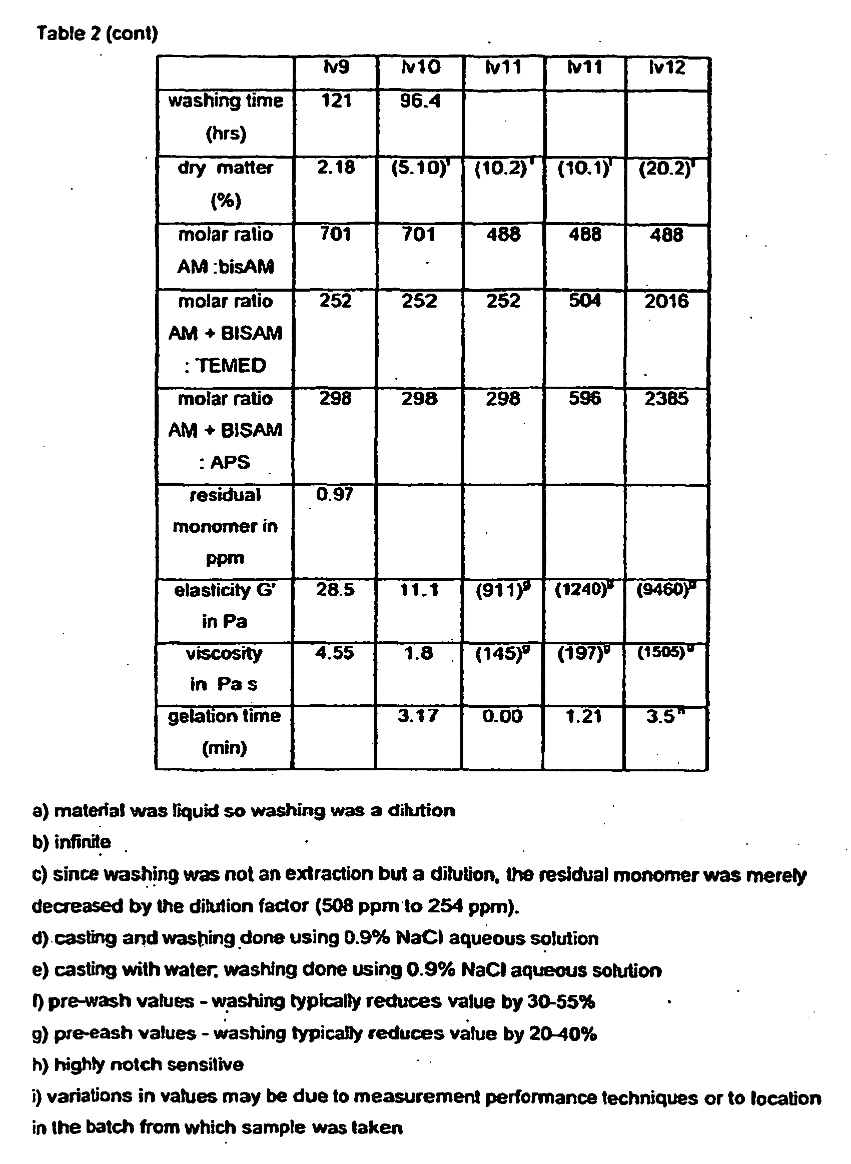

**Table 3:**

| Process parameters and features of resulting gel: medium viscosity formulations | | | | | |
|---|---|---|---|---|---|
| | mv1 | mv2 | mv3 | mv4 | mv5 |
| washing time (hrs) | 97 | 211.5 | 96 | 94.8 | 90.3 |
| dry matter (%) | 3.14 | 2.49 | 3.25 | 3.29 | 3.22 |
| molar ratio AM :bisAM | 310 | 310 | 290 | 289 | 289 |
| molar ratio AM + BISAM :TEMED | 252 | 252 | 252 | 251 | 252 |
| molar ratio AM + BISAM : APS | 299 | 299 | 299 | 299 | 299 |
| residual monomer in ppm | 1.6 | | 1.5 | | |
| elasticity G' in Pa | 108.5 | | 129 | 133.5 | |
| viscosity in Pas | 17.4 | | 20.6 | 21.30 | |
| gelation time (min) | 2.5 | 2.5 | 2.18 | | |

**Table 4:**

| Process parameters and features of resulting gel: high viscosity formulations | | | | | |
|---|---|---|---|---|---|
| | hv1 | hv2 | hv3 | hv4 | hv5 |
| washing time (hrs) | 119.5 | 516 | 122 | 95.5 | 116.7 |
| dry matter (%) | 3.47 | 2.5 | 3.56 | 3.83 | 3.42 |
| molar ratio AM :bisAM | 260 | 260 | 260 | 260 | 260 |
| molar ratio AM + BISAM : TEMED | 315 | 315 | 604 | 313 | 314 |
| molar ratio AM + BISAM :APS | 376 | 376 | 755 | 375 | 376 |
| residual monomer in ppm | 0.2 | | | | |
| elasticity G' in Pa | 343 | 274 | | 314.5 | |
| viscosity in Pas | 54.7 | 43.65 | | 50.1 | |
| gelation time (min) | 2.18 | 2.18 | 7.5 | | |

### Example 2

### Analysis of Hydrogel

### Characteristics of the gel

The washed hydrogels will have typical properties outlined below.

**Table 5**

| Property | Low viscosity version | High viscosity version |
|---|---|---|
| Cross-linking density | 0.25% | 0.40% |
| Swelling rate | 80-120% | 50-70% |
| Rheol. elasticity modulus, G^{*\} | 10-80 Pa | 250-700 Pa |
| Rheol. Complex viscosity, η* | 2-10 Pas | 50-90 Pas |
| Dry matter | 1,6-3% | 3-5% |
| Refractive index | 1,335-1,338 | 1.338-1.340 |

### Example 3

Swelling occurs during wash procedure and will typically show a swelling profile as shown in below Figures 2 and 3.

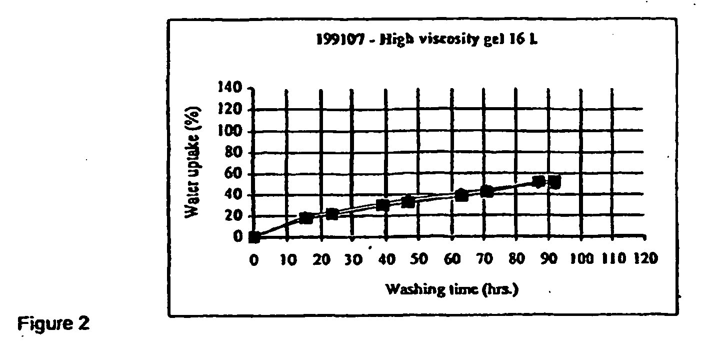

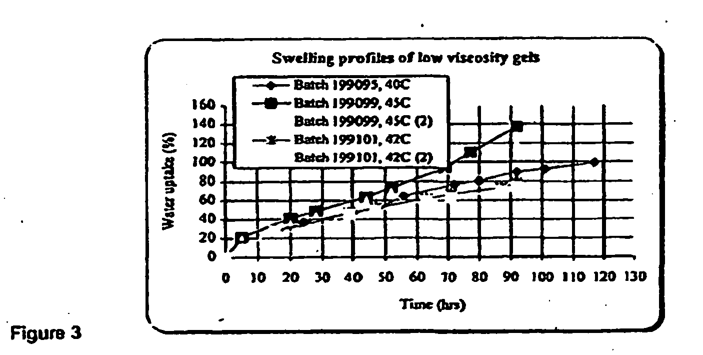

### Example 4

### Method of Administration for Body Contouring

### Treatment by injection

a) The injection of the gel may be performed under local anaesthesia.
b) The procedure must be performed under sterile conditions. Pharmaceuticals are not to be injected into the gel
c) The gel is pre-filled in sterile syringes of 1 mL with luer-lock and should be injected subcutaneously with a thin gauge needle, e.g 27 G. Needles should be CE-marked.
d) Inject the necessary amount of the gel subcutaneously in a retrograde manner by injecting the gel while withdrawing the needle. A patient record label is part of the packaging and is removable and to be attached to the patient record to ensure that the product is traceable.
e) After the injection is performed, a light manipulation may be performed in order to obtain an even or desired distribution of the gel. The injected gel will form a stable, soft part in the connective tissue and will give long lasting cosmetically satisfactory appearance.
f) Further injection sessions may be performed to achieve the desired affect.

### Example 5

### Method of Administration for Body Contouring

### Treatment by injection

a) The injection of the gel may be performed under local anaesthesia.
b) The procedure must be performed under sterile conditions. Pharmaceuticals are not to be injected into the gel
c) The get is pro-filled in sterile syringes of 1 mL with luer-lock and should be injected subcutaneously with a thin gauge needle, e.g 27 G. Needles should be CE-marked.
d) Inject the necessary amount of the gel subcutaneously in a retrograde manner by injecting the gel white withdrawing the needle. A patient record label is part of the packaging and is removable and to be attached to the patient record to ensure that the product is traceable.
e) After the injection is performed, a light manipulation may be performed in order to obtain an even or desired distribution of the gel. The injected gel will form a stable, soft part in the connective tissue and will give long lasting cosmetically satisfactory appearance.
f) Further injection sessions may be performed to achieve the desired affect.

### Example 6

### Method of Administration for soft Tissue Filling

a) The injection of the gel may be performed under local anaesthesia, but for correction of wrinkles and folds, local anaesthesia is not necessarily required. For lip augmentation, anaesthesia through the nerve block is recommended.
b) The procedure must be performed under sterile conditions. Pharmaceutical are not to be injected into the gel
c) The gel is pre-filled in sterile syringes of 1 mL with luer-tock and should be injected subcutaneously with a thin gauge needle, e.g 27 G. Needles should be CE-marked.
d) Inject the necessary amount of the gel subcutaneously in a retrograde manner by injecting the gel while withdrawing the needle. A patient record label is part of the packaging and is removable and to be attached to the patient record to ensure that the product is traceable.
e) After the injection is performed, a light manipulation may be performed in order to obtain an even or desired distribution of the gel. The injected gel will form a stable, soft part in the connective tissue and will give long lasting cosmetically satisfactory appearance.
f) Further injection sessions may be performed to achieve the desired affect.

### Post-operative procedures

If oedema occurs, ice packs may be applied locally Exposure to direct sunlight or extreme cold or heat is advised until the initial swelling and redness has been resolved.

### Adverse Events/Side Effects

It is not uncommon for patients to develop some pain within the first 2-3 days postoperatively. A mild degree of oedema will occur in some patients during the first 2-3 days after injection.

The correct injection technique Is crucial for the final result of the treatment and to be performed by authorised personnel.

The gel is sterilised (such as by moist heat or autoclavation). In the even that the package is damaged or opened but unused, sterility may be compromised and the contents should be discarded. Re-sterilisation is not advisable.

### Example 7

### Clinical Experience for Soft Tissue Filling

1) Approximately 900 patients underwent facial corrections with the gel. The overall cosmetic results were excellent and the frequency or adverse events was 0.02% (Kovanskaya V.A.; Scientific conference, 13-16 October 2000).
2) A total of 150 adults undergoing correction of the contour deformities of the face were treated with the injectable gel. The amount of gel injected was from 0.2 to 11 mL.

Scheduled visits took place at a screening day (3 days prior to day 0), day 0 (first injection), day 7, day 28. month 3, month 6 and at end of study visit month 12 and underwent a physical examination and vital signs test, pregnancy test, blood and serum analysis, hematology test, immunology test, urine analysis, concomitant treatment, side effect and events analysis, cosmetic outcome analysis by patient and surgeon as well as completing a questionnaire according to the schedule of Table 6.

### Results

The overall rating of the outcome of the surgery was from very good to good from both the patients and the surgeon. In some instances, the patients wanted to continue the treatment and receive further injections. Several surgeons remarked spontaneously on the questionnaire that the patients were happy with the result and that the gel was easy to handle and administer.

The gel was very well tolerated. Only a few side-effects were recorded and the Adverse Events oedema and inflammation were reported by the patient. The Adverse Events resolved spontaneously after a few days.

**Table 6**

| | Screening (at least -3 days) | Day 0 (Facial correction) | | Day 7 ±1 day | Day 28 ±2 days | month 3 ±7 days | month 6 ±7 days | month 12 ±7 days |
|---|---|---|---|---|---|---|---|---|
| | | pre-op | post-op | | | | | |
| information / informed consent | x | | | | | | | |
| physical examination | | x | | x | x | x | x | x |
| vital signs | | x | | | | | x | x |
| pregnancy. x test | | | | | | | | |
| blood and serum analysts | sampling | x | | | | | x | x |
| Haematology | sampling | x | | | | | x | x |
| Immunology | sampling | x | | | | | x | x |
| Urine analysis | | x | | | | | x | x |
| Concomitant treatment | | x | x | x | x | x | x | x |
| side effects and events | | | x | x | x | x | x | x |
| questionnaire (comptaints) | | | x | x | x | x | x | x |
| Cosmetic outcome Patient Surgeon | | | | | | | | |
| | | | | | x x | x x | x x | x x |

## Claims

1. A biocompatible hydrogel obtainable by
combining acrylamide and methylene bis-acrylamlde in a molar ratio of 225:1 to 500: 1; radical Initiation; and washing with pyrogen-free water or saline solution for 80 to 100 hours;
so as to give at least 0.5% by weight polyacrylamide and less than 3.5% by weight polyacrylamide, based on the total weight of the hydrogel;
for use as an injectable endoprosthesis for soft tissue filling by injection into a mammal.

2. The hydrogel according to claim 1 for use in the treatment of a cosmetic or functional defect of the face by injection, wherein the hydrogel has an elasticity module from 35 to 480 Pa.

3. The hydrogel according to claim 1 for use in the treatment of a cosmetic or functional defect in lips by injection, wherein the hydrogel has an elasticity module from 35 to 480 Pa.

4. The hydrogel according to claim 1 for use in the preparation of an endoprosthesis for body contouring.

5. The hydrogel according to claim 2, wherein the endoprosthesis is for use in correction of facial contour deformities due to ageing, acne, trauma, surgery, infection or congenital deformities.

6. The hydrogel according to claim 2, wherein the endoprosthesis is for use in correction of the cheekbones, nasolabial folds, glabellar frowns, depressed contours of the mouth or the chin, to treat wrinkles, folds, facial asymmetries, or altering the shape of a nose

7. The hydrogel according to claim 3, wherein endoprosthesis is for correcting the size or shape the lips.

8. The hydrogel according to any one of the preceding claims, wherein the endoprothesis is for use in correcting aesthetic defects caused by congenital, traumatic or ageing alterations.

9. The hydrogel according to any one of the preceding claims comprising at least 95% pyrogen-free water or saline solution.

10. The hydrogel according to any one of the preceding claims comprising at least 1% polyacrylamide by weight, based on the total weight of the hydrogel.

11. The hydrogel according to any one of the preceding claims having a concentration of acrylamide and N,N'-methylene-bis-acrylamide below 50 ppm.

12. The hydrogel according to any of the preceding claims further comprising cells to allow for cellular engraftment.

13. The hydrogel according to claim 12, wherein the cells are stem cells.

## Patentansprüche

1. Biokompatibles Hydrogel, erhältlich durch
das Vereinen von Acrylamid und Methylen-bis-acrylamid in einem molaren Verhältnis von 225:1 bis 500:1; Radikalkettenstart und Waschen mit pyrogenfreiern Wasser oder Kochsalzlösung über einen Zeitraum von 80 bis 100 Stunden;
um mindestens 0,5 Gew.-% Polyacrylamid und weniger als 3,5 Gew.-% Polyacrylamid, bezogen auf das Gewicht des Hydrogels, zu ergeben;
zur Verwendung als injizierbare Endoprothese zum Füllen von Weichteilgewebe durch Injektion bei einem Säugetier.

2. Hydrogel nach Anspruch 1 zur Verwendung bei der Behandlung eines kosmetischen oder funktionellen Defekts des Gesichts durch Injektion, wobei das Hydrogel ein Elastizitätsmodul von 35 bis 480 Pa aufweist.

3. Hydrogel nach Anspruch 1 zur Verwendung bei der Behandlung eines kosmetischen oder funktionellen Defekts der Lippen durch Injektion, wobei das Hydrogel ein Elastizitätsmodul von 35 bis 480 Pa aufweist.

4. Hydrogel nach Anspruch 1 zur Verwendung bei der Herstellung einer Endoprothese für die Körperformung.

5. Hydrogel nach Anspruch 2, wobei die Endoprothese zur Verwendung bei der Korrektur von Deformitäten der Gesichtsform aufgrund von Alter, Akne, Trauma, einem operativen Eingriff, Infektion oder von angeborenen Deformitäten dient.

6. Hydrogel nach Anspruch 2, wobei die Endoprothese zur Verwendung bei der Korrektur von Wangenknochen, nasolabialen Falten, Glabellafalten, tiefe Konturen an Mund oder Kinn, der Behandlung von Runzeln, Falten, Gesichtsasymmetrien oder der Veränderung der Nasenform dient.

7. Hydrogel nach Anspruch 3, wobei die Endoprothese zur Korrektur der Größe oder Form der Lippen dient.

8. Hydrogel nach einem der vorhergehenden Ansprüche, wobei die Endoprothese zur Verwendung bei der Korrektur ästhetischer Defekte dient, die durch angeborene, traumabedingte oder altersbedingte Veränderungen verursacht wurden.

9. Hydrogel nach einem der vorhergehenden Ansprüche, umfassend mindestens 95 % pyrogenfreies Wasser oder Kochsalzlösung.

10. Hydrogel nach einem der vorhergehenden Ansprüche, umfassend mindestens 1 Gew.-% Polyacrylamid, bezogen auf das Gesamtgewicht des Hydrogels.

11. Hydrogel nach einem der vorhergehenden Ansprüche mit einer Konzentration von Acrylamid und N,N'-Methylen-bis-acrylamid von weniger als 50 ppm.

12. Hydrogel nach einem der vorhergehenden Ansprüche, ferner umfassend Zellen, die eine Zelltransplantation ermöglichen.

13. Hydrogel nach Anspruch 12, wobei die Zellen Stammzellen sind.

## Revendications

1. Un hydrogel biocompatible pouvant être obtenu par
combinaison d'acrylamide et de méthylènebisacrylamide selon un rapport molaire de 225/1 à 500/1 ; initiation radicalaire ; et lavage avec de l'eau apyrogène ou une solution saline pendant 80 à 100 heures ;
de manière à produire au moins 0,5 % de polyacrylamide en poids et moins de 3,5 % de polyacrylamide en poids, sur la base du poids total de l'hydrogel ;
destiné à être utilisé comme endoprothèse injectable pour le comblement de tissus mous par injection chez un mammifère.

2. L'hydrogel selon la revendication 1 destiné à être utilisé pour le traitement d'un défaut cosmétique ou fonctionnel de la face par injection, où l'hydrogel présente un module d'élasticité de 35 à 480 Pa.

3. L'hydrogel selon la revendication 1 destiné à être utilisé pour le traitement d'un défaut cosmétique ou fonctionnel des lèvres par injection, où l'hydrogel présente un module d'élasticité de 35 à 480 Pa.

4. L'hydrogel selon la revendication 1 destiné à être utilisé dans la préparation d'une endoprothèse pour le remodelage du corps.

5. L'hydrogel selon la revendication 2, où l'endoprothèse est destinée à être utilisée pour la correction de déformations du contour du visage causées par l'âge, l'acné, un trauma, la chirurgie, une infection ou une malformation congénitale.

6. L'hydrogel selon la revendication 2, où l'endoprothèse est destinée à être utilisée pour corriger les pommettes, les sillons nasogéniens, les rides de la glabelle, un affaissement des contours de la bouche ou du menton, pour traiter des rides, des plis, des asymétries faciales ou pour modifier la forme d'un nez.

7. L'hydrogel selon la revendication 3, où l'endoprothèse est destinée à corriger la taille ou la forme des lèvres.

8. L'hydrogel selon l'une quelconque des revendications précédentes, où l'endoprothèse est destinée à être utilisée pour corriger des défauts esthétiques causés par des altérations congénitales, traumatiques ou dues au vieillissement.

9. L'hydrogel selon l'une quelconque des revendications précédentes comprenant au moins 95 % d'eau apyrogène ou de solution saline.

10. L'hydrogel selon l'une quelconque des revendications précédentes comprenant au moins 1 % de polyacrylamide en poids, sur la base du poids total de l'hydrogel.

11. L'hydrogel selon l'une quelconque des revendications précédentes présentant une concentration d'acrylamide et de N,N'-méthylènebisacrylamide inférieure à 50 ppm.

12. L'hydrogel selon l'une quelconque des revendications précédentes comprenant également des cellules destinées à permettre une greffe cellulaire.

13. L'hydrogel selon la revendication 12, où les cellules sont des cellules souches.
